# EUROPEAN PATENT SPECIFICATION

(11) **EP 3 097 895 B1**
(45) Date of publication and mention of the grant of the patent: **17.01.2018**
(21) Application number: 16170631.2
(22) Date of filing: 20.05.2016
(51) Int. Cl.: A61F 13/15

(54) **METHOD FOR FORMING UNBONDED FIBROUS STRUCTURE**
VERFAHREN ZUR HERSTELLUNG NICHT VERBUNDENER FASERSTRUKTUREN
PROCÉDÉ DE FORMATION D'UNE STRUCTURE FIBREUSE NON LIÉE

(30) Priority: 28.05.2015 EP 15169526
(43) Date of publication of application: 30.11.2016
(73) Proprietor: The Procter & Gamble Company, Cincinnati, OH 45202 (US)
(72) Inventor: VAN DER KLUGT, Walter Pieter Hendrik Laurentius, 53881 Euskirchen (DE); TOMBUELT-MEYER, Thomas, 53881 Euskirchen (DE); TRINKAUS, Jan Michael, 53881 Euskirchen (DE)
(74) Representative: Mather, Peter Geoffrey

(56) References cited:
- EP-A1- 0 478 182
- EP-A1- 2 508 156
- WO-A1-2013/143783
- WO-A2-2013/143728
- US-A- 3 973 291
- US-A1- 2004 089 516
- US-A1- 2013 270 067

## Description

### BACKGROUND OF THE INVENTION

The use of fibrous structures in absorbent articles such as diapers or feminine hygienic articles is well known in the art. Fibrous structures may be either consolidated, bonded webs, such as nonwoven webs, but can also be unbonded structures, often made of natural fibers such as cellulose fibers or chemically modified cellulose fibers.

It is known to cut discrete lengths of a material web and to apply the cut material web to a carrier.

US 6,811,019, issued on November 2nd 2004, discloses a method and apparatus utilizing servomotors for placing parts onto a moving web. This patent discloses the cutting of a material web into discrete parts, and applying the discrete parts onto a carrier.

Different methods for forming and/or transferring an unbonded fibrous web to a carrier are disclosed in EP 0 478 182, US 3,973,291, EP 2 508 156, US 2004/089516 and US 2013/270067.

Unbonded fibrous structures can for example be used as absorbent cores, wherein the fibers are typically mixed with superabsorbent gelling materials, such as superabsorbent polymer particles. Moreover, such unbonded fibrous structures can be used in so-called liquid acquisition systems overlaying the absorbent core.

With the processes used today for high speed manufacture of fibrous webs for absorbent articles, unbonded fibrous webs are typically formed on relatively large diameter drums. Such drums enable a variety of different sized fibrous webs to be manufactured on the same drum diameter, but at different patch lengths and different pitch. However a smaller drum would be more energy efficient, and would enable more efficient use of space.

There remains a need for transforming fibrous material into an unbonded fibrous structure on high speed manufacturing lines. In particular it would be desirable to provide fibrous structures on a drum of fixed diameter, whilst retaining the flexibility to provide different patch length and/or different pitch of fibrous structures to meet needs of different sized absorbent articles. Moreover it would be desirable to have the capability to take these said different patch lengths, and combine these at different spacings to the carrier web, all without distortion of the shape and contour of the unbonded fibrous structure.

### SUMMARY OF THE INVENTION

The present invention relates to a method for forming and transferring an unbonded fibrous structure to a carrier, the method comprising the steps of:
receiving fibrous material on a shell travelling at a first speed through a receiving zone to form a fibrous patch;
transferring the shell from the receiving zone to an application zone;
applying the fibrous web to the carrier travelling at a second speed through the application zone; and
controlling the speed of the shell by a motor, the motor comprising a stator and at least one mover, wherein the stator (103) comprises a plurality of stationary electromagnets and wherein the movers (60) comprise permanent magnets and wherein the variable speed of the movers (60) along the stationary track (100) is controlled by varying the current supplied to the stationary electromagnets;
wherein the shell is driven by at least one mover, wherein the mover maintains the shell at the first speed in the receiving zone and the second speed in the application zone, and wherein the mover is mounted on a stationary track.

### BRIEF DESCRIPTION OF THE DRAWINGS

FIG. 1 schematically shows a front perspective view of an exemplary apparatus of the present invention;
FIG. 2 schematically shows another front perspective view of the apparatus in FIG. 1;
FIG. 3 schematically shows a rear perspective view of the apparatus in FIG. 1.

### DETAILED DESCRIPTION OF THE INVENTION

Programmable servomotors have been used in manufacturing processes and offer, amongst other advantages, the possibility to position individual components relative to one another, and to accelerate or decelerate the speed of individual components in the process. Rotary and linear programmable servomotor systems are known.

More recently a modular, fully integrated linear motor with power electronics and displacement measurements have been combined into a single device. WO2013/143728 and WO2013/143783, both published on October 3^{rd} 2013, describe a linear transport system comprising a stator device and a position detection device for detecting a position of a movable element in a drive device. The manufacturer, Beckhoff Automation GmbH, markets such a system under the name "eXtended Transport System", or XTS. A similar system is offered by Rockwell Automation, which is marketed under the name "i-Trac".

The present invention applies the principle of programmable servomotors to the method of forming unbonded fibrous structures, such as patches of fibrous material, and transferring the fibrous patch to a carrier, preferably a carrier web. Unbonded fibrous structures have no inherent strength or integrity and hence cannot be formed using existing process technology such as disclosed in US 6,811,019.

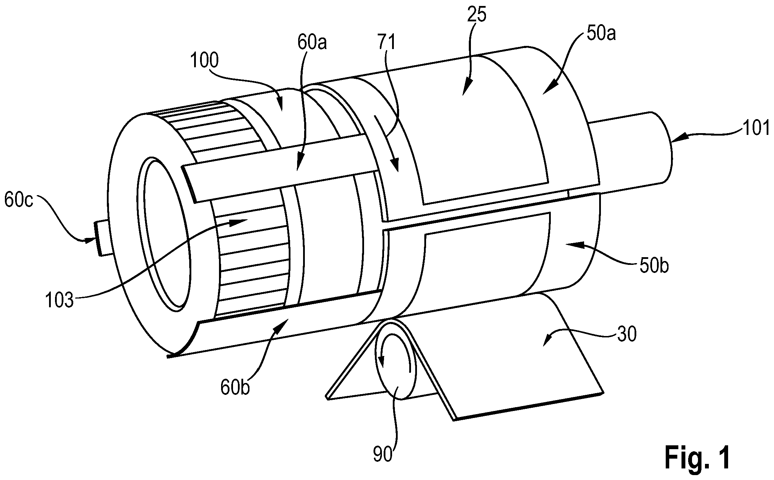

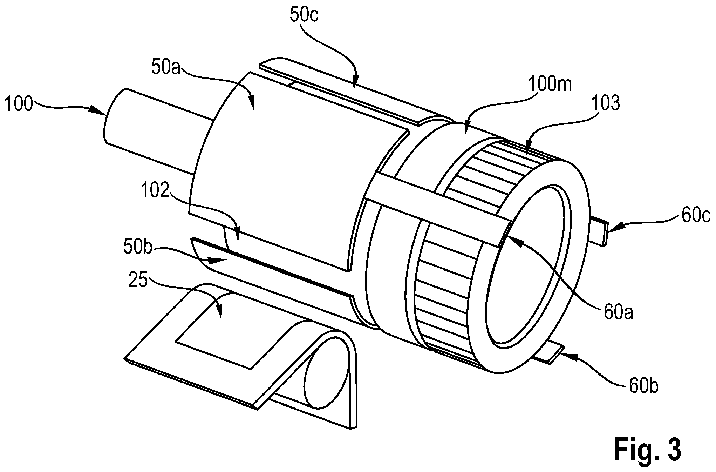

FIG. 1-3 depicts a non-limiting exemplary embodiment of the method for receiving fibrous material on a shell travelling at a first speed through a receiving zone 23 in a direction indicated by arrow 21, forming on the shell a fibrous patch 25 from the fibrous material, and then applying the fibrous patch 25 to a carrier 30 traveling at a second speed in a direction indicated by arrow 31. Carrier 30 may include, but is not limited to, a web substrate, belt, drum, and external-discrete part.

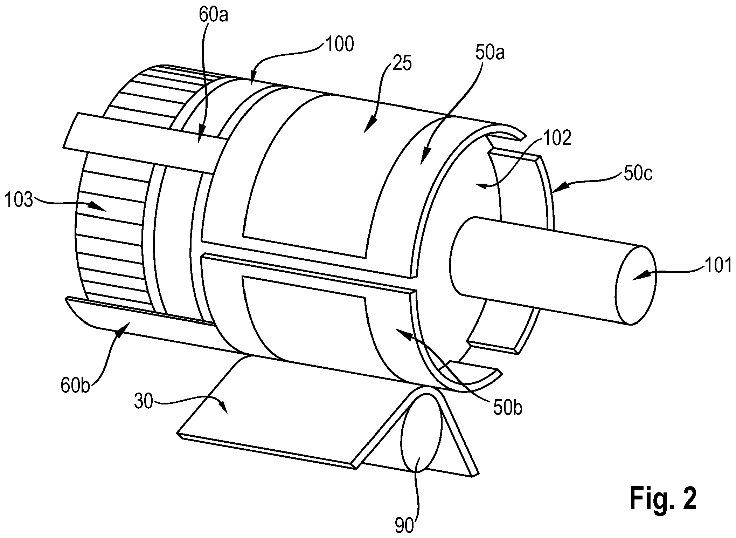

In this non-limiting exemplary embodiment of apparatus 10, three shells 50a-c are used, however, one skilled in the art would appreciate that any reasonable number of multiple shells may be used. Shells 50a-c are adjoined to a corresponding programmable motor by means of movers 60a-c that transport said shell along a stationary track 100 in a direction as indicated by arrow 71. In this exemplary embodiment, shells 50a-c have an axial length from about 4 cm to about 200 cm, tangential width from about 0.5 cm to about 200 cm, and a thickness from about 0.25 mm to about 300 mm. Shells 50a-c may be constructed of plastic, aluminum, steel, fibre reinforced composites, such as carbon fibre or glass fibre reinforced composites, and combinations thereof; although one skilled in the art would appreciate that other suitable materials may be used. The dimensions of the shell 50 may vary depending upon the desired output of the apparatus 10 and the size and shape of the discrete patches 25 being transferred. The shells 50 are driven around a stationary track 100 by a motor which comprises movers 60 and stator 103. The stator comprises programmable magnets. The shells 50 travel in the direction indicated by the arrow 71 as shown in FIG. 1. The circumferential, outer peripheral surface of the shell 50 defined by an outer radius, travels along and defines a path (e.g. an orbital path) that passes through a receiving zone 23 and an application zone 27. The receiving zone 23 and the application zone 27 are defined by the respective regions of the path traveled by the shell 50. The size and shape of the shells 50 may vary. For example, if the apparatus 10 includes three shells 50a-c, as illustrated in FIGS. 1 and 2, each shell 50 may have an outer peripheral arc length which spans from about 2 to about 115 degrees of the orbital path.

The use of a programmable motor in apparatus 10 provides an inexpensive and adaptable method for forming patches 25 at a speed and applying the patches 25 to a carrier 30 traveling at a different speed. The variable angular velocity of movers 60 is produced by varying the current supplied to electromagnets. Since the shell 50 is coupled to the output of the motor 60, changes in the angular velocity and position of the movers 60a-c directly correlate to changes in the angular velocity and position of shell 50. The current supplied to the electromagnets can be controlled using any of a variety of a methods for programming motors known to those skilled in the art such as, standard cam curve functions, a reference data table containing reference points, desired motor encoder points, and the like or combinations thereof.

The means of supplying the rotational movement required can be achieved in a plurality of methods to those skilled in the art. The programmable electric motors can be driven from any known power source that is capable of delivering a modulated signal such that the motor torque can be varied proportionally.

One or more power sources deliver a modulated torque signal. The torque signal is typically an electrical current which may be fed to the individual electromagnets by separate power supplies or by a single power supply and controlled by a plurality of methods to those skilled in the art.

As compared to conventional methods for changing the speed of a discrete part so that it can be applied to a continuously moving carrier (such as a slip-and-cut known in the art), the use of programmable motors provides the ability to obtain greater changes in speed and to maintain constant speeds for a fixed duration. The fixed speed dwell achieved by programmable motors can be accurately and quickly generated to control the length and placement of the parts. The use of programmable motors provides the ability to change the profile at will without requiring the fabrication of new parts.

The actual position of the shell 50 can be controlled by a plurality of methods known to those skilled in the art including, but not limited to, a position transducer (e.g., encoder-based system, resolver-based system, etc). For instance, the actual position of the shell 50 can be controlled by a programmable system that incorporates a position feedback from the shell 50 and mover 60. Alternatively, the actual position may not be needed if the position of the shell 50 can be inferred by other means known to those skilled in the art. Irrespective of the type of control method used, the control method may be used to provide the proportional signal to the motor power supplies that will generate the modulated torque signal. A control system may or may not be integrated into the motor power supply. A control system, along with the motor power supply, may or may not be integrated into the motor 103 itself. A control system may or may not be digitally controlled, and may be constructed in various methods, and configurations known to those skilled in the art. The control system, power supplies, feedback devices, and motor devices, and any other components required for the purpose of providing rotational movement are hereafter referred to as the "drive system" for shells 50.

The drive system (not shown) may be capable of continuously controlling the position of the shell 50, and allowing the shell to stay in phase to a given position on the recipient product, web, or host machine. The drive system may be capable of following speed transitions or position variations on the recipient product or web, by phasing itself, when necessary, to the recipient product, web, or host machine, with or without operator intervention. The drive system will allow for the registration of the discrete patch 25 on shell 50 in relation to the carrier 30, either upstream or downstream of shell 50.

The drive system may be capable of providing for a plurality of control methods and algorithms known to those skilled in the art for the purpose of providing motion and position control that will allow the transfer of a discrete patch 25 to a recipient product or web. The drive system may be capable of changing the part length with or without operator intervention, for the purpose of varying product sizes or continuous part length, or position variation control. The motion trajectory for shell 50 may be based on a pre-calculated profile or a profile that is modified by the speed of the recipient product or web.

Motor 60, 103 may be a programmable motor selected from the group consisting of a motor having a hollow shaft, a linear motor having a stationary track rail, a motor having a rotatable outer rotor and a stationary inner stator, and a motor having a rotor rotatable around a stationary component of a motor. In an exemplary embodiment, the movers 60 (e.g., cross bars running on the track) serve as the rotor (i.e., spins) and the outer portion serves as the stator 103 (i.e., stationary). In this exemplary embodiment, each single shell 50 is driven by a mover 60 and thus support shell 50 on one of its longitudinal ends; although one skilled in the art would appreciate that other arrangements of motor(s) to shell(s) may be used.

In a preferred embodiment the motor 60, 103 is a stationary series of electromagnets 103 where the switching of the magnets is programmable. This system is completed by movers 60a-c that have permanent magnets, and are mounted to a track 100. The placement of both types of magnets is such that interaction between the magnetic fields is possible such that a driving force can be created. The shells 50a-c are attached to the movers by suitable means. The track 100 can have different shapes, provided the profile of the track creates a continuous and closed path in two, or even in three, dimensions. A preferred profile is circular in shape as shown in FIGS. 1-3.

Shells 50a-c may further include a gripping mechanism so that the outer concave surface of the shell can capture a discrete fibrous patch 25 in the receiving zone 23 and transport it to the application zone 27. Said gripping mechanism may be vacuum, electrostatic forces, mechanical forces (e.g., clamps) or another suitable method known in the art. In this exemplary embodiment, shells 50a-c may be air pervious such that vacuum may be applied by a roll 80 which is positioned interior to said shells. A vacuum supply line 101 draws vacuum from the air plenum within the stator 103. The vacuum force helps to keep discrete fibrous patches 25 positioned against shells 50a-c, or can aid its formation as is common in air laid structures. Vacuum pressure may be applied over the entire circumference of roll 80 or it may also be selectively applied from the receiving zone 23 to the application zone 27. Appropriate means to prevent loss of vacuum force when the shells are opened up need to be applied.

The shells 50a-c supported in a cantilevered manner over stator 103, while the drive mechanism sits to the opposite side of the vacuum body. In such embodiment the vacuum supply can be coming from the side not obstructed by the motors.

Discrete fibrous patches 25 are applied to carrier 30 in the application zone 27. Preferably the carrier 30 is a carrier web, such as a nonwoven web. The carrier 30 is conveyed through the application zone 27. At such time, it may be desirable to discontinue the vacuum force. A backing roll 90 may be used to help bring carrier 30 in close proximity to application zone 27 for transferring of discrete fibrous patches 25 to carrier 30. Other known techniques to assist the transferring of discrete fibrous patches 25 to carrier 30 may be appreciated by one skilled in the art including, but not limited to, adhesive applied on discrete fibrous patch 25, adhesive applied on carrier 30, electrostatic charge between the discrete fibrous patch 25 and carrier 30, vacuum on the carrier 30, blown air to blow discrete fibrous patch 25 off of shell 50, and the like or combinations thereof. Alternately, the transfer can include the generation of a weld between the discrete fibrous patch 25 and the carrier 30 by any of a variety of means known to those skilled in the art including, but not limited to, pressure generation at a nip formed between the shell 50 and backing roll 90 at transfer, interaction between a pattern on the shell 50 and an ultrasonic horn behind the carrier 30 at transfer, and the like, or combinations thereof. In addition, in order to aid the welding process, the discrete fibrous patch 25 may be modified on the shell 50 by energy addition using any mechanism known to those skilled in the art including, but not limited to, hot air currents, ultraviolet lighting, laser bombardment and the like or combinations thereof.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A method for forming and transferring an unbonded fibrous structure to a carrier, the method comprising the steps of:
receiving fibrous material on a shell (50) travelling at a first speed through a receiving zone, to form a fibrous patch (25);
transferring the shell (50) from the receiving zone (23) to an application zone (27);
applying the fibrous patch (25) to the carrier (30) travelling at a second speed through the application zone (27); and
controlling the speed of the shell by a motor, the motor comprising a stator (103) and at least one mover (60), wherein the stator (103) comprises a plurality of stationary electromagnets and wherein the movers (60) comprise permanent magnets and wherein the variable speed of the movers (60) along the stationary track (100) is controlled by varying the current supplied to the stationary electromagnets;
wherein the shell is driven by at least one mover (60), wherein the mover (60) maintains the shell (50) at the first speed in the receiving zone (23) and the second speed in the application zone (27), and wherein the mover (60) is mounted on a stationary track (100).

2. The method according to claim 1 wherein the stationary track (100) follows a continuous, closed path.

3. The method according to claim 2 wherein the closed, continuous path is a circular path.

4. The method according to any of claims 1 or 2 wherein the fibrous material is held against the shell, at least in the receiving zone (23) by vacuum.

5. The method according to any of claims 1 to 4 wherein a plurality of shells travel at the first speed through the receiving zone (23) and at a second speed through the application zone (27), and wherein the speed of each shell (50) is independently controlled by at least one mover (60).

## Patentansprüche

1. Verfahren zum Formen einer ungebundenen Faserstruktur und Übertragen derselben auf einen Träger, wobei das Verfahren die folgenden Schritte umfasst:
Aufnahme von Fasermaterial auf einer Schale (50), die sich mit einer ersten Geschwindigkeit durch einen Aufnahmebereich bewegt, um ein Faserstück (25) zu bilden:
Übertragen der Schale (50) von dem Aufnahmebereich (23) auf einen Anwendungsbereich (27);
Auftragen des Faserstücks (25) auf den Träger (30), der sich mit einer zweiten Geschwindigkeit durch den Anwendungsbereich (27) bewegt; und
Steuern der Geschwindigkeit der Schale durch einen Motor, wobei der Motor einen Stator (103) und mindestens ein Antriebselement (60) umfasst, wobei der Stator (103) eine Vielzahl von stationären Elektromagneten umfasst, und wobei die Antriebselemente (60) Permanentmagnete umfassen, und wobei die variable Geschwindigkeit der Antriebselemente (60) entlang der stationären Bahn (100) durch Variieren des an die stationären Elektromagnete gelieferten Stromes gesteuert wird;
wobei die Schale durch mindestens ein Antriebselement (60) angetrieben wird, wobei das Antriebselement (60) in dem Aufnahmebereich (23) die erste Geschwindigkeit der Schale (50) und in dem Anwendungsbereich (27) die zweite Geschwindigkeit der Schale aufrecht erhält, und wobei das Antriebselement (60) an einer stationären Bahn (100) angebracht ist.

2. Verfahren nach Anspruch 1, wobei die stationäre Bahn (100) einer ununterbrochenen, geschlossenen Strecke folgt.

3. Verfahren nach Anspruch 2, wobei die geschlossene ununterbrochene Strecke eine kreisförmige Strecke ist.

4. Verfahren nach einem der Ansprüche 1 oder 2, wobei das Fasermaterial durch ein Vakuum mindestens in dem Aufnahmebereich (23) gegen die Schale gehalten wird.

5. Verfahren nach einem der Ansprüche 1 bis 4, wobei sich eine Vielzahl von Schalen mit einer ersten Geschwindigkeit durch den Aufnahmebereich (23) und mit einer zweiten Geschwindigkeit durch den Anwendungsbereich (27) bewegt, und wobei die Geschwindigkeit von jeder Schale (50) unabhängig voneinander durch mindestens ein Antriebselement (60) gesteuert wird.

## Revendications

1. Procédé de formation et de transfert d'une structure fibreuse non liée sur un support, le procédé comprenant les étapes consistant à :
recevoir un matériau fibreux sur une coque (50) se déplaçant à une première vitesse à travers une zone de réception, pour former une pièce fibreuse (25) ;
transférer la coque (50) de la zone de réception (23) vers une zone d'application (27) ;
appliquer la pièce fibreuse (25) au support (30) se déplaçant à une deuxième vitesse à travers la zone d'application (27) ; et
commander la vitesse de la coque par un moteur, le moteur comprenant un stator (103) et au moins un dispositif d'entraînement (60), dans lequel le stator (103) comprend une pluralité d'électro-aimants stationnaires et dans lequel les dispositifs d'entraînement (60) comprennent des aimants permanents et dans lequel la vitesse variable des dispositifs d'entraînement (60) le long de la piste stationnaire (100) est commandée en faisant varier le courant fourni aux électro-aimants stationnaires ;
dans lequel la coque est entraînée par au moins un dispositif d'entraînement (60), dans lequel le dispositif d'entraînement (60) maintient la coque (50) à la première vitesse dans la zone de réception (23) et à la deuxième vitesse dans la zone d'application (27), et dans lequel le dispositif d'entraînement (60) est monté sur une piste stationnaire (100).

2. Procédé selon la revendication 1, dans lequel la piste stationnaire (100) suit une trajectoire fermée continue.

3. Procédé selon la revendication 2, dans lequel la trajectoire fermée continue est une trajectoire circulaire.

4. Procédé selon l'une quelconque des revendications 1 ou 2, dans lequel le matériau fibreux est maintenu contre la coque, au moins dans la zone de réception (23), par le vide.

5. Procédé selon l'une quelconque des revendications 1 à 4, dans lequel une pluralité de coques se déplace à la première vitesse à travers la zone de réception (23) et à une deuxième vitesse à travers la zone d'application (27), et dans lequel la vitesse de chaque coque (50) est indépendamment commandée par au moins un dispositif d'entraînement (60).
